# EUROPEAN PATENT APPLICATION

(11) **EP 2 327 437 A1**
(43) Date of publication of application: **01.06.2011**
(21) Application number: 10189349.3
(22) Date of filing: 29.10.2010
(51) Int. Cl.: A61M 5/168, A61M 5/14, A61M 5/142

(54) **Infusion pump module and infusion system**

(30) Priority: 25.11.2009 JP 2009267723; 08.09.2010 JP 2010201089
(71) Applicant: Ricoh Company Ltd., Tokyo 143-8555 (JP)
(72) Inventor: Sugimoto, Yasunori, Tokyo 143-8555 (JP); Kamada, Teruki, Tokyo 143-8555 (JP); Tomoyama, Takashi, Tokyo 143-8555 (JP)
(74) Representative: Schwabe - Sandmair - Marx

(57) **Abstract**

An infusion pump module (10) includes a body case (20) that includes an inflow channel (23) for a liquid medicine; a first connecting flow channel (24); a second connecting flow channel (25); and an outflow channel (27) ; an auxiliary case (30) joined to the body case (20) and includes an auxiliary flow channel (31) connected to the first and second connecting flow channel (25); a flow sensor (40) that forms a measurement flow channel (41) and measures a flow rate of the liquid medicine; an infusion pump (50) that forms a transfer flow channel (51) connected to the inflow channel (23) and the first connecting flow channel (24), and discharges the liquid medicine by a piezoelectric element (55); a gasket (60) that seals each connection portion between the inflow channel (23) and the transfer flow channel (51) and so on; and a cover case (80) fastened to the body case (20) to fix the gasket (60), the infusion pump (50), and the flow sensor (40) to the body case (20).

## Description

### CROSS-REFERENCE TO RELATED APPLICATIONS

The present application claims priority to and incorporates by reference the entire contents of Japanese Patent Application No. 2009-267723 filed in Japan on November 25, 2009 and Japanese Patent Application No. 2010-201089 filed in Japan on September 8, 2010.

### BACKGROUND OF THE INVENTION

### 1. Field of the Invention

The present invention relates to an infusion pump module and an infusion system.

### 2. Description of the Related Art

Conventionally, when a liquid medicine is injected into a patient in a medical setting or the like, a liquid transfer pump has been used that includes a pump using a piezoelectric element and an oscillator circuit for driving the piezoelectric element (see, for example, Japanese Patent Application Laid-open No. H8-303352). In such a liquid transfer pump, the oscillator circuit applies a voltage of a predetermined frequency to oscillate (drive) the piezoelectric element, and the pump transfers a liquid inside a flow channel along with the oscillation of the piezoelectric element.

In the liquid transfer pump as described above, it is possible to control the amount of a liquid medicine to be transferred by arranging a flow sensor on a liquid delivery vessel, such as a tube, connected to the liquid transfer pump, and is possible to control the oscillator circuit depending on a measurement result obtained by the flow sensor.

However, in the above configuration, because it is necessary to separately prepare the liquid transfer pump, the liquid delivery vessel, and the flow sensor and then make up a system by connecting each of these devices, the system itself becomes large, resulting in limited installable locations.

The present invention has been made in view of the above, and it is an object of the present invention to provide an infusion pump module and an infusion system that are compact and capable of controlling the amount of a liquid medicine to be transferred.

### SUMMARY OF THE INVENTION

It is an object of the present invention to at least partially solve the problems in the conventional technology.

According to an aspect of the present invention, there is provided an infusion pump module including: a body case (20) that includes an inflow channel (23) having an inflow port for a liquid medicine; a first connecting flow channel (24); a second connecting flow channel (25); and an outflow channel (27) having an outflow port (26) for the liquid medicine; an auxiliary case (30) joined to the body case (20) and that includes an auxiliary flow channel (31) connected to the first connecting flow channel (24) and the second connecting flow channel (25); a flow sensor (40) that forms a measurement flow channel (41) connected to the second connecting flow channel (25) and the outflow channel (27), and measures a flow rate of the liquid medicine flowing through the measurement flow channel (41); an infusion pump (50) that forms a transfer flow channel (51) connected to the inflow channel (23) and the first connecting flow channel (24), and discharges the liquid medicine flowed into the transfer flow channel (51) from the inflow channel (23) to the first connecting flow channel (24) by using a piezoelectric element (55); a gasket (60) that seals a connection portion between the inflow channel (23) and the transfer flow channel (51), a connection portion between the transfer flow channel (51) and the first connecting flow channel (24), a connection portion between the second connecting flow channel (25) and the measurement flow channel (41), and a connection portion between the measurement flow channel (41) and the outflow channel (27); and a cover case (80) that is fastened to the body case (20) to thereby fix the gasket (60), the infusion pump (50), and the flow sensor (40) to the body case (20).

According to another aspect of the present invention, there is provided an infusion system including: the infusion pump module mentioned above; and a system controller that controls oscillation of the piezoelectric element (55) depending on a measurement result of the flow rate obtained by the flow sensor (40), and controls the amount of the liquid medicine to be transferred by the infusion pump (50).

The above and other objects, features, advantages and technical and industrial significance of this invention will be better understood by reading the following detailed description of presently preferred embodiments of the invention, when considered in connection with the accompanying drawings.

### BRIEF DESCRIPTION OF THE DRAWINGS

Fig. 1 is a perspective view of a general configuration example of an infusion pump module according to an embodiment;
Fig. 2 is a cross-sectional view taken along II-II of Fig. 1;
Fig. 3 is a schematic diagram of a general configuration example of an infusion system according to the embodiment; and
Fig. 4 is a perspective view of a general configuration example of an infusion pump module according to a modified example.

### DETAILED DESCRIPTION OF THE PREFERRED EMBODIMENTS

Exemplary embodiments of an infusion pump module and an infusion system according to the present invention are explained in detail below with reference to the accompanying drawings.

The configuration of the infusion pump module according to an embodiment of the present invention is explained below.

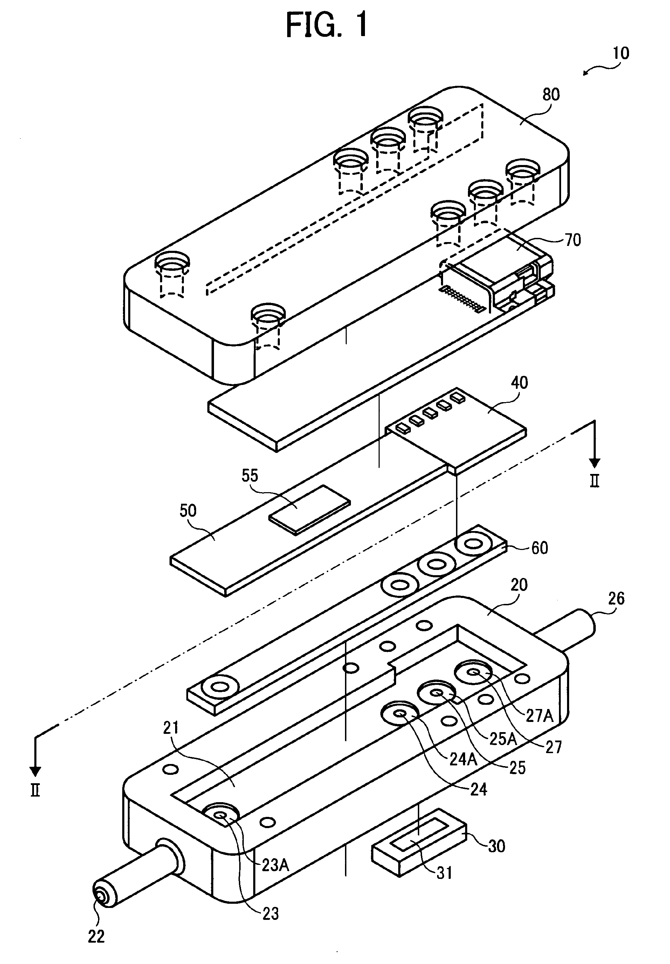

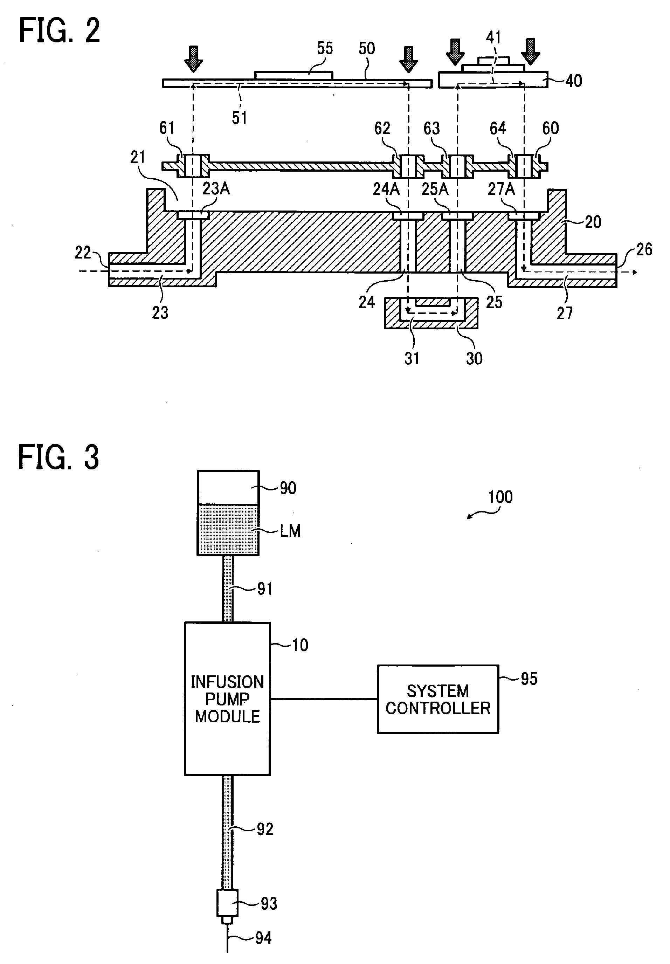

Fig. 1 is a perspective view of a general configuration example of an infusion pump module 10 according to the embodiment. As illustrated in Fig. 1, the infusion pump module 10 of the embodiment includes a body case 20, an auxiliary case 30, a flow sensor 40, an infusion pump 50, a gasket 60, a PCB (Printed Circuit Board) 70, and a cover case 80. Fig. 2 is a cross-sectional view taken along II-II of Fig. 1. In the cross-sectional view of Fig. 2, side views of the infusion pump 50 and the flow sensor 40 are also illustrated. The general configuration of the infusion pump module 10 is explained below with reference to Figs. 1 and 2. Dashed arrows in Fig. 2 illustrate flow channels for flowing a liquid medicine inside the infusion pump module 10, and bold arrows in Fig. 2 illustrate a direction of load applied by fastening the body case 20 and the cover case 80.

The body case 20 is an approximately rectangular case, and includes a concave portion 21 for housing the infusion pump 50 and the flow sensor 40 in the approximate center of the top surface thereof. On the body case 20 are formed an inflow channel 23 having an inflow port 22 for a liquid medicine, a first connecting flow channel 24, a second connecting flow channel 25, and an outflow channel 27 having an outflow port 26 for a liquid medicine in this order from the front surface of the body case 20 in the longitudinal direction. The body case 20 is a resin case molded by injection molding.

The inflow port 22 is formed in the center bottom portion of the front surface of the body case 20 in the longitudinal direction. The inflow channel 23 is a bent flow channel bent by approximately 90 degrees, and connects the inflow port 22 and the bottom surface of the concave portion 21. The first connecting flow channel 24 and the second connecting flow channel 25 are linear flow channels piercing through the body case 20 in the thickness direction (in the vertical direction), and connect the bottom surface of the concave portion 21 and the bottom surface of the body case 20. The outflow port 26 is arranged in the center bottom portion of the rear surface of the body case 20 in the longitudinal direction. The outflow channel 27 is a bent flow channel bent by approximately 90 degrees, and connects the bottom surface of the concave portion 21 and the outflow port 26. Circular grooves 23A, 24A, 25A, 27A for engaging the gasket 60 to be described later are formed on the periphery of the inflow channel 23, the first connecting flow channel 24, the second connecting flow channel 25, and the outflow channel 27, respectively, on the bottom surface of the concave portion 21.

The auxiliary case 30 is a rectangular resin case smaller than the body case 20, and includes a U-shaped auxiliary flow channel 31 to be connected to the first connecting flow channel 24 and the second connecting flow channel 25. The auxiliary case 30 is joined to the bottom surface of the body case 20 with a solvent such that the auxiliary flow channel 31 matches the first connecting flow channel 24 and the second connecting flow channel 25. The solvent used for joining the auxiliary case 30 and the body case 20 volatizes after the joining, so that it is ensured that components of the solvent are not mixed into the liquid medicine flowing through the first connecting flow channel 24, the auxiliary flow channel 31, and the second connecting flow channel 25.

The flow sensor 40 constitutes an inverted U-shaped measurement flow channel 41 connected to the second connecting flow channel 25 and the outflow channel 27, and measures the flow rate of the liquid medicine flowing through the measurement flow channel 41. For example, a thermal mass sensor can be used as the flow sensor 40. The flow sensor 40 is housed in a space on the rear surface side of the concave portion 21 in the longitudinal direction so as to sandwich the gasket 60 to be described later between itself and the concave portion 21.

The infusion pump 50 constitutes an inverted U-shaped transfer flow channel 51 connected both to the inflow channel 23 and the first connecting flow channel 24. Furthermore, the infusion pump 50 includes a piezoelectric element 55, and discharges the liquid medicine flowed into the transfer flow channel 51 from the inflow channel 23 to the first connecting flow channel 24 due to oscillation of the piezoelectric element 55. The liquid medicine, discharged out to the first connecting flow channel 24, flows through the auxiliary flow channel 31, the second connecting flow channel 25, and the measurement flow channel 41 so as to be discharged from the outflow channel 27. Each of the flow channels of the embodiment is in the form of a tube. The infusion pump 50 is housed in a space on the front surface side of the concave portion 21 in the longitudinal direction so as to sandwich the gasket 60 to be described later between itself and the concave portion 21. That is, according to the embodiment, the infusion pump 50 and the flow sensor 40 are placed side by side in the longitudinal direction (hosed in the concave portion 21).

The gasket 60 is a rectangular sealing member placed in the concave portion 21 of the body case 20. According to the embodiment, a silicone rubber sheet is used as the gasket 60. The gasket 60 includes projections 61 to 64 to be respectively engaged with the grooves 23A to 27A formed on the bottom surface of the concave portion 21. The central portion of each of the projections 61 to 64 is hollowed out in the shape of a circle, so that a through hole is formed. The gasket 60 is placed on the body case 20 (the concave portion 21) such that the projections 61 to 64 are respectively engaged with the grooves 23A to 27A, and is moderately squashed and deformed by placing thereon the infusion pump 50 and the flow sensor 40. Consequently, the gasket 60 seals a connection portion between the inflow channel 23 and the transfer flow channel 51, a connection portion between the transfer flow channel 51 and the first connecting flow channel 24, a connection portion between the second connecting flow channel 25 and the measurement flow channel 41, and a connection portion between the measurement flow channel 41 and the outflow channel 27, so that leakage and infiltration of the liquid medicine can be prevented.

The PCB 70 transmits drive power from an external apparatus to the piezoelectric element 55 included in the infusion pump 50 and the flow sensor 40. More specifically, respective electrodes included in the PCB 70, the piezoelectric element 55, and the flow sensor 40 are connected to one another with a probe or the like (not illustrated), so that the PCB 70 transmits the drive power from the external apparatus to the piezoelectric element 55 and the flow sensor 40. Furthermore, the PCB 70 transmits a measurement result obtained by the flow sensor 40 to an external apparatus, and oscillates the piezoelectric element 55 by applying a voltage of a predetermined frequency to the piezoelectric element 55.

The cover case 80 is fastened to the body case 20 with a predetermined fastening member such as a screw. Consequently, the components of the infusion pump module 10, such as the flow sensor 40, the infusion pump 50, the gasket 60, and the PCB 70, are assembled onto the body case 20 and fixed between the body case 20 and the cover case 80. Specifically, according to the embodiment, the cover case 80 and the body case 20 are fastened to each other at a front and a rear positions in the short-side direction of each of the connection portion between the inflow channel 23 and the transfer flow channel 51, the connection portion between the transfer flow channel 51 and the first connecting flow channel 24, the connection portion between the second connecting flow channel 25 and the measurement flow channel 41, and the connection portion between the measurement flow channel 41 and the outflow channel 27. Therefore, as illustrated in Fig. 2, load for pressing the connection portions of the flow channels downward is applied, so that the infusion pump 50 and the flow sensor 40 are assembled onto the body case 20 such that the gasket 60 are compressed at the connection portions of the flow channels. Therefore, leakage of the liquid medicine can infallibly be prevented.

As described above, according to the infusion pump module 10 of the embodiment, the infusion pump 50 and the flow sensor 40 are housed in the body case 20 in which the flow channels are formed, so that a single flow channel can be formed. Therefore, it is possible to provide an infusion pump module that is compact and capable of controlling the amount of a liquid medicine to be transferred.

Furthermore, according to the infusion pump module 10 of the embodiment, because the flow sensor 40 is used that measures the flow rate by flowing the liquid medicine inside thereof, it is possible to reduce costs compared to use of a flow sensor that measures the flow rate from the outside. Therefore, the infusion pump module can become suitable for disposables.

Moreover, according to the infusion pump module 10 of the embodiment, because the configuration is simple in which the infusion pump 50 and the flow sensor 40 are placed side by side in the longitudinal direction, it is possible to flexibly handle design changes. When the infusion pump 50 and the flow sensor 40 are placed side by side, a U-shaped flow channel is necessary to transfer an infusion from the infusion pump 50 to the flow sensor 40. However, because the body case 20 is molded by injection molding, it is difficult to form the U-shaped flow channel in the body case 20. To cope with this, in the infusion pump module 10 according to the embodiment, the auxiliary case 30 including the U-shaped auxiliary flow channel 31 is joined to the body case 20.

Furthermore, the infusion pump module 10 of the embodiment is formed such that all of the components can be assembled in one direction (e.g., from the top to the bottom). Therefore, it is possible to improve the assemblability and to simplify assembling equipments. Specifically, according to the embodiment, because a rectangular silicone rubber sheet is used as the gasket 60, the positioning of the gasket can be easy and the number of components can be reduced compared to use of an O-ring or the like. Therefore, the assembling can be made easier.

Moreover, according to the infusion pump module 10 of the embodiment, a method such as boding or welding is not applied for joining the components and preventing fluid leakage, whereas the joining is performed with solvent and the fluid leakage is prevented by using the gasket. Therefore, according to the embodiment, it is ensured that the components of an adhesive agent are not mixed into a liquid medicine flowing through each flow channel. Specifically, according to the embodiment, solvent sealing is applied between the components, such as the body case 20 and the auxiliary case 30, for which the sealing with solvent is suitable. Whereas the gasket is used between the components, such as between the infusion pump 50 and the body case 20 or between the flow sensor 40 and the body case 20, for which the solvent sealing is unsuitable. Therefore, it is possible to reduce the number of the gaskets. Here, because the infusion pump 50 is a silicone wafer, it is difficult to ensure the adhesiveness by the solvent sealing and it is possible that a sealed portion may be broken due to oscillation during the time of driving. Therefore, the infusion pump 50 is not suitable for the solvent sealing.

Next, the configuration of the infusion system according to the embodiment is explained.

Fig. 3 is a schematic diagram of a general configuration example of an infusion system 100 including the infusion pump module 10 described above. As illustrated in Fig. 3, the infusion system 100 of the embodiment includes the infusion pump module 10 and a system controller 95. The inflow port 22 of the infusion pump module 10 is connected, via a tube 91, to a container 90 containing a liquid medicine LM to be injected into a biological body. Furthermore, the outflow port 26 of the infusion pump module 10 is connected, via a tube 92, to an attachment 93 to which a needle 94 to be inserted into the biological body (blood vessel) is attached. A highly elastic and self-expandable flexible tube is used as each of the tubes 91 and 92.

By controlling the infusion pump module 10 by the infusion pump 50, the liquid medicine LM contained in the container 90 is caused to flow a flow channel formed of the tube 91, the infusion pump module 10, the tube 92, and the needle 94.

The system controller 95 controls the infusion pump module 10, and may be realized by a microcomputer or the like. The system controller 95 is electrically connected to the PCB 70 in the infusion pump module 10, and receives the measurement result of the flow rate of the liquid medicine LM from the flow sensor 40 via the PCB 70. The system controller 95 controls the oscillation of the piezoelectric element 55 included in the infusion pump 50 depending on the received measurement result to thereby control the amount of the liquid medicine to be transferred by the infusion pump 50. More specifically, the system controller 95 adjusts at least one of a voltage value and a frequency of a voltage pulse to be applied to the piezoelectric element 55 included in the infusion pump 50 so that the flow rate of the liquid medicine LM matches a target amount by using the received measurement result.

The system controller 95 further includes an interface such as an operation panel (not illustrated) for allowing an operator to input the (target) amount of a liquid medicine to be injected, a (target) injection time, and the like, a display panel (not illustrated) for displaying the injection state of the liquid medicine LM, and an alarm device (not illustrated) for notifying abnormality in the injection state.

As described above, according to the infusion system 100 of the embodiment, the infusion pump 50 and the flow sensor 40 are housed in the infusion pump module 10, and the infusion pump 50 can adjust the transfer flow rate by feeding the measurement result back from the flow sensor 40. Therefore, it is possible to realize the infusion system using a compact pump module with reduced flow error.

### Modified Example:

The present invention is not limited to the above embodiments, and can be modified in various forms.

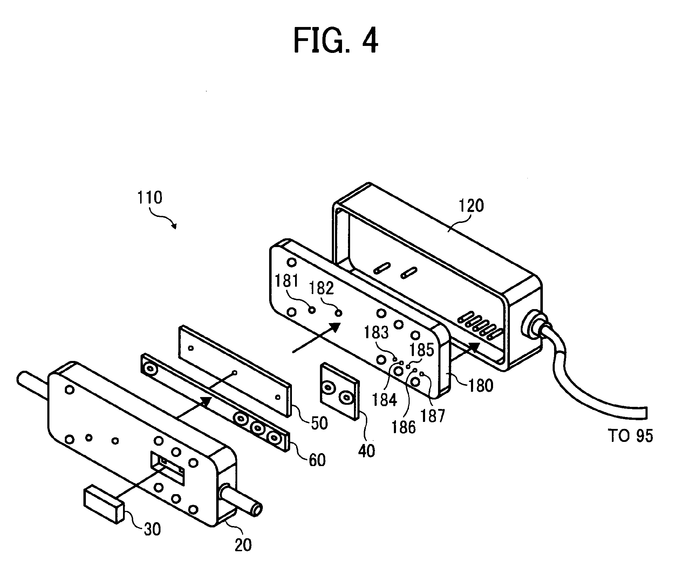

In the embodiment described above, an example is explained in which the PCB is housed in the infusion pump module. However, it is possible not to include the PCB as inside the case of an infusion pump module 110 illustrated in Fig. 4. In this case, as illustrated in Fig. 4, it is desirable to establish electrical connections between the infusion pump 50 and the system controller 95 and between the flow sensor 40 and the system controller 95 via through holes 181 to 187 formed on an under cover case 180, by using a harness 120 communicating with the system controller 95. With this configuration, the number of components of the infusion pump module 110 can be reduced, enabling to further downsize the infusion pump module 110. Furthermore, even when the infusion pump module 110 is used as the disposable, the PCB need not be disposed, so that environmental load can be reduced with low costs.

Furthermore, in the embodiment described above, the bent portion of each flow channel is not specially processed. However, the bent portion of each flow channel can be curved. Consequently, it is possible to prevent the liquid medicine from being pooled.

According to an embodiment of the present invention, it is possible to provide an infusion pump module and an infusion system that are compact and capable of controlling the amount of a liquid medicine to be transferred.

Although the invention has been described with respect to specific embodiments for a complete and clear disclosure, the appended claims are not to be thus limited but are to be construed as embodying all modifications and alternative constructions that may occur to one skilled in the art that fairly fall within the basic teaching herein set forth.

## Claims

1. An infusion pump module (10) comprising:
a body case (20) that includes
an inflow channel (23) having an inflow port for a liquid medicine;
a first connecting flow channel (24);
a second connecting flow channel (25); and
an outflow channel (27) having an outflow port (26) for the liquid medicine;
an auxiliary case (30) joined to the body case (20) and that includes
an auxiliary flow channel (31) connected to the first connecting flow channel (24) and the second connecting flow channel (25);
a flow sensor (40) that forms a measurement flow channel (41) connected to the second connecting flow channel (25) and the outflow channel (27), and measures a flow rate of the liquid medicine flowing through the measurement flow channel (41);
an infusion pump (50) that forms a transfer flow channel (51) connected to the inflow channel (23) and the first connecting flow channel (24), and discharges the liquid medicine flowed into the transfer flow channel (51) from the inflow channel (23) to the first connecting flow channel (24) by using a piezoelectric element (55);
a gasket (60) that seals a connection portion between the inflow channel (23) and the transfer flow channel (51), a connection portion between the transfer flow channel (51) and the first connecting flow channel (24), a connection portion between the second connecting flow channel (25) and the measurement flow channel (41), and a connection portion between the measurement flow channel (41) and the outflow channel (27); and
a cover case (80) that is fastened to the body case (20) to thereby fix the gasket (60), the infusion pump (50), and the flow sensor (40) to the body case (20).

2. The infusion pump module (10) according to claim 1, wherein
the infusion pump (50) discharges the liquid medicine to the first connecting flow channel (24) by oscillating the piezoelectric element (55).

3. The infusion pump module (10) according to claim 1 or 2, wherein
the auxiliary flow channel (31) has a U-shape.

4. The infusion pump module (10) according to any one of claims 1 to 3, wherein
the infusion pump (50) and the flow sensor (40) are placed side by side in a longitudinal direction of the infusion pump module (10).

5. The infusion pump module (10) according to any one of claims 1 to 4, wherein
the body case (20) and the auxiliary case (30) are made of resin, and
the auxiliary case (30) is joined to the body case (20) with solvent.

6. The infusion pump module (10) according to any one of claims 1 to 5, wherein
the gasket (60) is a silicone rubber sheet on which through holes are formed at positions corresponding to the respective connection portions.

7. The infusion pump module (10) according to any one of claims 1 to 6, further comprising:
a printed circuit board (70) that transmits drive power to the piezoelectric element (55) and the flow sensor (40), wherein
the cover case (80) is fastened to the body case (20) to thereby fix the gasket (60), the infusion pump (50), the flow sensor (40), and the printed circuit board (70) to the body case (20).

8. An infusion system comprising:
the infusion pump module (10) according to any one of claims 1 to 7; and
a system controller that controls oscillation of the piezoelectric element (55) depending on a measurement result of the flow rate obtained by the flow sensor (40), and controls the amount of the liquid medicine to be transferred by the infusion pump (50).
